# EUROPEAN PATENT APPLICATION

(11) **EP 4 434 488 A1**
(43) Date of publication of application: **25.09.2024**
(21) Application number: 23799241.7
(22) Date of filing: 28.04.2023
(51) Int. Cl.: A61C 8/00, A61C 9/00

(54) **SCANNING APPARATUS, CONNECTING METHOD AND APPARATUS THEREFOR, ELECTRONIC DEVICE, AND MEDIUM**

(30) Priority: 02.05.2022 CN 202210477086
(71) Applicant: Shining 3D Tech Co., Ltd., Hangzhou, Zhejiang 311258 (CN)
(72) Inventor: CHEN, Xiaojun, Hangzhou, Zhejiang 311258 (CN); MA, Chao, Hangzhou, Zhejiang 311258 (CN); ZHANG, Wei, Hangzhou, Zhejiang 311258 (CN); ZHAO, Xiaobo, Hangzhou, Zhejiang 311258 (CN)
(74) Representative: Grünecker Patent- und Rechtsanwälte PartG mbB
(86) International application number: PCT/CN2023/091812
(87) International publication number: WO 2023/213255

(57) **Abstract**

Embodiments of the present disclosure relate to a scanning apparatus, a connection method thereof, an apparatus, an electronic device, and a medium. The scanning apparatus includes a scanning body and an auxiliary feature body connected with the scanning body. Auxiliary feature points are arranged on the scanning body and/or the auxiliary feature body. Adopting the above scanning apparatus simplifies a using mode, requires less external assistance, and can improve scanning and implanting accuracy during positioning based on coordinate information of the auxiliary feature points, thereby further increasing a utilization rate of an intraoral scanner in implant cases.

## Description

### Cross-Reference to Related Application

The present disclosure claims the priority of Chinese Patent Application No. 202210477086.1, filed with China National Intellectual Property Administration on May 2, 2022, and entitled "SCANNING APPARATUS, CONNECTION METHOD FOR THE SCANNING APPARATUS, APPARATUS, ELECTRONIC DEVICE, AND MEDIUM", which is incorporated in its entirety herein by reference.

### Technical Field

The present disclosure relates to the technical field of intraoral scanning, and in particular to a scanning apparatus, a connection method for the scanning apparatus, an apparatus, an electronic device, and a medium.

### Background

Typically, in scanning and repairing scenarios, relevant target positions are determined through scanning.

In the related art, due to the limitation of the scanning range, a multi-data stitching solution is usually used during data scanning. Due to the existence of cumulative errors, the overall accuracy of a model is ultimately not high.

### Summary

### (I). To-be-solved technical problems

The present disclosure aims to solve the technical problem that in an existing multi-data stitching solution, due to cumulative errors, the overall accuracy of a model is not high.

### (II). Technical solutions

In order to solve the above technical problem, the embodiments of the present disclosure provide a scanning apparatus, a connection method for the scanning apparatus, an apparatus, an electronic device, and a medium.

In a first aspect, an embodiment of the present disclosure provides a scanning apparatus. The scanning apparatus includes:
a scanning body; and
an auxiliary feature body connected with the scanning body,
where auxiliary feature points are arranged on the scanning body and/or the auxiliary feature body.

In a second aspect, an embodiment of the present disclosure provides a connection method for the scanning apparatus. The method includes:
obtaining fixed distances between scanning bodies within an oral cavity region; and
determining target auxiliary feature bodies with target lengths based on the fixed distances so as to connect the scanning bodies based on the target auxiliary feature bodies within the oral cavity region.

An embodiment of the present disclosure further provides a connection apparatus for the scanning apparatus. The apparatus includes:
a distance obtaining component, configured to obtain fixed distances between scanning bodies within an oral cavity region; and
a determination component, configured to determine target auxiliary feature bodies with target lengths based on the fixed distances so as to connect the scanning bodies based on the target auxiliary feature bodies within the oral cavity region.

In a third aspect, an embodiment of the present disclosure further provides an electronic device. The electronic device includes: a processor; and a memory configured to store executable instructions of the processor. The processor is configured to read the executable instructions from the memory and execute the instructions to implement the connection method for the scanning apparatus provided by the embodiments of the present disclosure.

In a fourth aspect, an embodiment of the present disclosure further provides a computer-readable storage medium. The storage medium has a computer program stored therein. The computer program is configured to execute the connection method for the scanning apparatus provided by the embodiments of the present disclosure.

It should be understood that the above general description and the following detailed description are only exemplary and explanatory, and cannot limit the present disclosure.

### Brief Description of Figures

Accompanying drawings herein are incorporated into the specification to form a part of the specification, illustrate embodiments conforming to the present disclosure, and are used for explaining the principle of the present disclosure together with the specification.

In order to describe technical solutions in embodiments of the present disclosure or in the prior art more clearly, the accompanying drawings required to be used in descriptions of the embodiments or the prior art will be briefly introduced below, and it is apparent that those of ordinary skill in the art can obtain other accompanying drawings according to these accompanying drawings without creative work.
FIG. 1 is a schematic structural diagram of a scanning apparatus according to an embodiment of the present disclosure;
FIG. 2 is an example diagram of connection of a scanning apparatus according to an embodiment of the present disclosure;
FIG. 3a is an example diagram of connection of another scanning apparatus according to an embodiment of the present disclosure;
FIG. 3b is an example diagram of connection of still another scanning apparatus according to an embodiment of the present disclosure;
FIG. 4a is a structural example diagram of a scanning apparatus according to an embodiment of the present disclosure;
FIG. 4b is a structural example diagram of another scanning apparatus according to an embodiment of the present disclosure;
FIG. 4c is a structural example diagram of still another scanning apparatus according to an embodiment of the present disclosure;
FIG. 4d is a structural example diagram of yet another scanning apparatus according to an embodiment of the present disclosure;
FIG. 5 is a schematic flowchart of a connection method for a scanning apparatus according to an embodiment of the present disclosure;
FIG. 6a is a schematic diagram of a connection scenario for a scanning apparatus according to an embodiment of the present disclosure;
FIG. 6b is a schematic diagram of a connection scenario for another scanning apparatus according to an embodiment of the present disclosure;
FIG. 7 is a schematic structural diagram of a connection apparatus for a scanning apparatus according to an embodiment of the present disclosure; and
FIG. 8 is a schematic structural diagram of an electronic device according to an embodiment of the present disclosure.

### Detailed Description

To make objectives, technical solutions, and advantages of embodiments of the present disclosure clearer, the technical solutions in the embodiments of the present disclosure are clearly and completely described as below, and it is apparent that the described embodiments are a part rather all of embodiments of the present disclosure. Based on the embodiments of the present disclosure, all other embodiments obtained by those of ordinary skill in art without creative work shall fall within the scope of protection of the present disclosure.

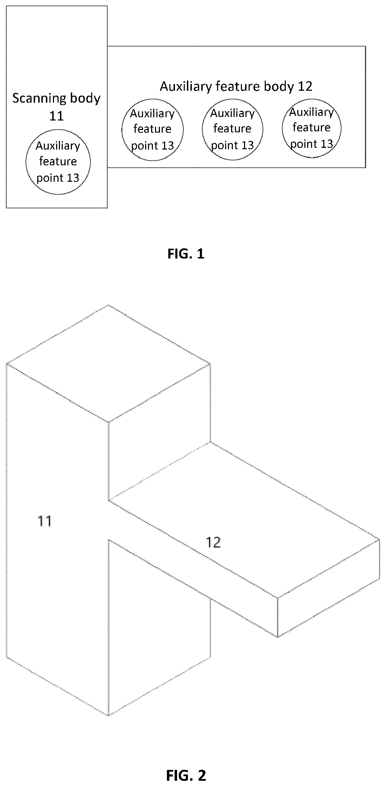

FIG. 1 is a schematic structural diagram of a scanning apparatus according to an embodiment of the present disclosure. As shown in FIG. 1, the scanning apparatus includes:
a scanning body 11 and an auxiliary feature body 12 connected with the scanning body 11. Auxiliary feature points 13 are arranged on the scanning body 11 and/or the auxiliary feature body 12.

In the embodiment of the present disclosure, by arranging the auxiliary feature body 12 connected with the scanning body 11, a plurality of scanning apparatuses may be connected through connection between the auxiliary feature bodies 12, thereby improving the accuracy of intraoral scanning. Additionally, the auxiliary feature points 13 are arranged on the scanning body 11 and/or the auxiliary feature body 12 so as to perform positioning based on coordinate information of the auxiliary feature points 13, thereby improving the accuracy of intraoral scanning and implantation, and further increasing a utilization rate of the scanning apparatus.

In the embodiment of the present disclosure, the scanning body 11 may be a scan post body, or a prepared tooth, etc.

In some embodiments, the scanning body 11 is provided with an adjustable-angle connection structure connected with the auxiliary feature body 12.

An angle of the adjustable-angle connection structure may be selected and set according to an application scenario. For example, the scanning body 11 is perpendicular to the auxiliary feature body 12, or is fixedly connected with the auxiliary feature body by a certain tilt angle.

It should be noted that one or two sides of the scanning body 11 may be connected with the auxiliary feature body 12, which is specifically selected and set according to an application scenario.

Exemplarily, as shown in FIG. 2, the scanning body 11 and the auxiliary feature body 12 are vertically connected, and the auxiliary feature body 12 is a cuboid.

In some embodiments, the auxiliary feature body 12 is connected with the scanning body 11 through threads; and/or, the auxiliary feature body 12 is connected with the scanning body 11 through a latch; and/or, the auxiliary feature body 12 is fixedly connected with the scanning body 11 through a collar; and/or, the auxiliary feature body 12 is connected with the scanning body 11 through a mode of lap joint or magnetic attraction.

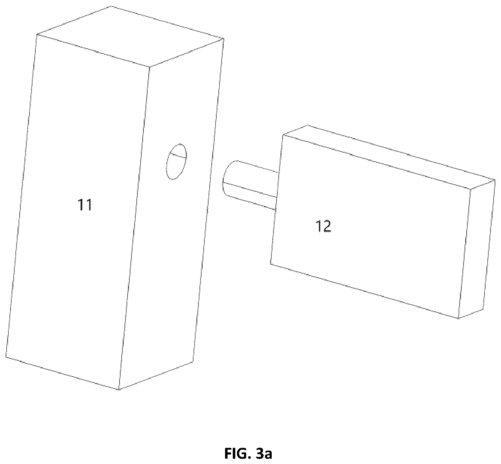

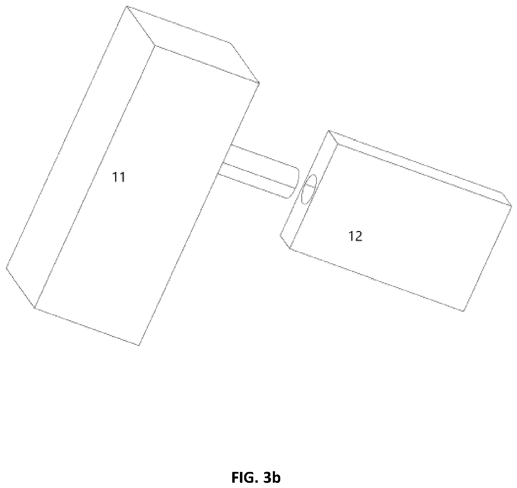

Exemplarily, as shown in FIG. 3a, the auxiliary feature body 12 is connected with the scanning body 11 through a latch; and as shown in FIG. 3b, the auxiliary feature body 12 is fixedly connected with the scanning body 11 through a collar.

In some embodiments, the auxiliary feature points 13 are spherical; and/or, codes; and/or, mark points; and/or, colors. The auxiliary feature points 13 may be in the shape of the auxiliary feature body itself.

Each auxiliary feature point 13 can uniquely identify one feature. That is, the auxiliary feature points 13 are arranged on the scanning body 11 and/or the auxiliary feature body 12. Each auxiliary feature point 13 can uniquely identify a corresponding position feature on the scanning body 11 and/or the auxiliary feature body 12. For example, a target feature a and a target feature b are respectively set at a position 1 on the scanning body 11 and a position 2 on the auxiliary feature body 12. The target feature a can uniquely identify the position feature of the position 1 on the scanning body 11, and the target feature b can uniquely identify the position feature of the position 2 on the auxiliary feature body 12.

It should be understood that different shapes, colors, two-dimensional codes, etc. on the scanning body 11 and/or the auxiliary feature body 12 that uniquely identify the corresponding position features on the scanning body 11 and/or the auxiliary feature body 12 may be taken as the auxiliary feature points 13.

In this embodiment of the present disclosure, the auxiliary feature points being spherical may be understood in a way that the auxiliary feature body 12 is set in a spherical shape. The auxiliary feature points being codes may be understood in a way that a two-dimensional code, etc. are printed on the auxiliary feature body 12. The auxiliary feature points being mark points may be understood in a way that shapes such as circles and triangles, and colors distinct from the auxiliary feature body 12 are printed on the auxiliary feature body 12. The auxiliary feature points being colors may be understood in a way that different colors are directly printed on the auxiliary feature body 12.

In some embodiments, the auxiliary feature body 12 is drum-shaped; and/or, rectangular; and/or, conical.

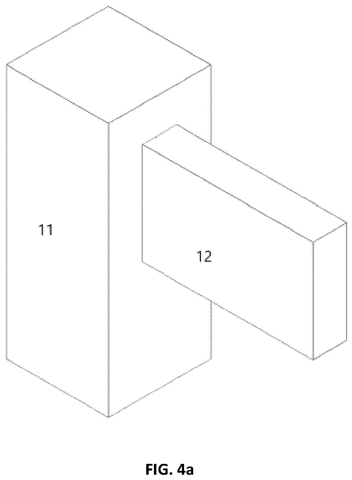

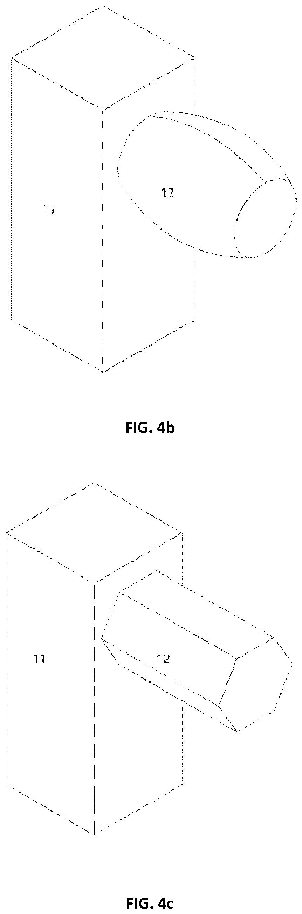

Exemplarily, as shown in FIG. 4a to FIG. 4c, auxiliary feature bodies 12 of different shapes further meet the needs of intraoral scanning in different scenarios.

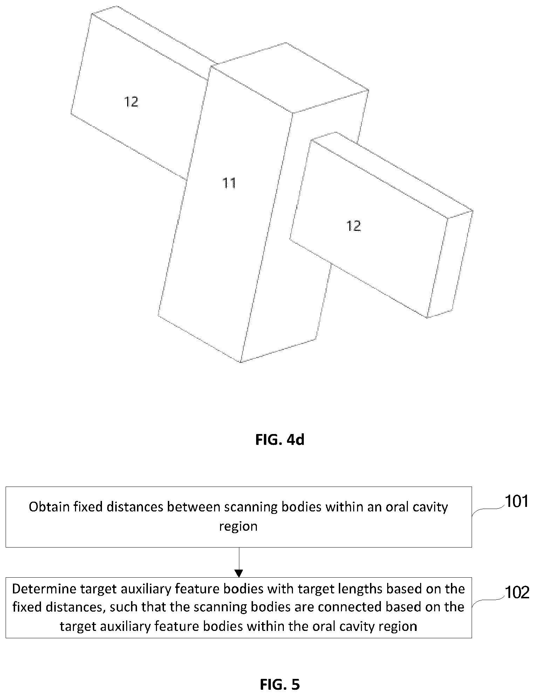

In this embodiment of the present disclosure, one scan post body 11 may also be connected with two auxiliary feature bodies 12. As shown in FIG. 4d, the scan post body 11 is connected with two rectangular auxiliary feature bodies 12.

In some embodiments, the scanning body 11 is provided with a non-anti-rotation structure connected with an implant.

In this embodiment of the present disclosure, the scanning body 11 and the implant are in non-anti-rotation connection. Therefore, the scanning body 11 may be controlled to rotate around an axis, and accordingly, when the auxiliary feature bodies 12 touch each other, a plurality of scanning bodies 11 can be connected by controlling the scanning body 11 to rotate around the axis through lap joint, overlapping, or other modes, thereby improving the accuracy of intraoral scanning.

In some embodiments, the auxiliary feature body 12 extends laterally outward from a scan post to be matched with the auxiliary feature body 12 on an adjacent intraoral scan post, allowing the target shape features 13 on the two auxiliary feature bodies 12 to be continuously distributed.

In some embodiments, two scanning bodies corresponding to two auxiliary feature bodies are connected by connecting the two auxiliary feature bodies. A distribution distance between the auxiliary feature points 13 set on the two auxiliary feature bodies is less than a preset distance threshold, which may be achieved by setting auxiliary feature bodies of different lengths or by using auxiliary feature bodies with lengths that can be stacked.

The auxiliary feature points 13 may be selected from one or more of spherical shapes, codes, and mark points according to the needs of an application scenario. The auxiliary feature points 13 are used for obtaining corresponding position coordinate points in an intraoral scanning process, thereby achieving accurate positioning of the scan post.

In this embodiment of the present disclosure, the accuracy of intraoral scanning and positioning can be further improved through coordinate information of the auxiliary feature points 13. Therefore, in the intraoral scanning process, images within an oral cavity from different perspectives may be obtained through a scanning window. To solve the problem of accuracy reduction caused by subsequent data stitching errors, it is necessary to control the distribution distance between the auxiliary feature points 13 to be less than the preset distance threshold during setting, thereby ensuring that the number of the auxiliary feature points 13 in the images obtained through the scanning window is greater than a preset quantity threshold, and guaranteeing the accuracy of intraoral scanning. The distance threshold and the quantity threshold may be adjusted in real time according to the needs of the application scenario for accuracy, which are not specifically limited in this embodiment of the present disclosure.

As an example scenario, a plurality of intraoral scan posts are installed in a target oral cavity. The intraoral scan post includes a scanning body used for being connected with an implant, and an auxiliary feature body connected with the scan post. Target features are arranged in the intraoral scan post. The target features are continuously distributed on the scan post and/or the auxiliary feature body. The target features are not confined to a single surface to be distributed on the scan post and/or the auxiliary feature body.

Specifically, an intraoral scanner scans the target oral cavity to obtain a plurality of frames of images, and transmits the images to a data processing component for data processing. The data processing component executes the following method:

A plurality of frames of images are obtained, initial three-dimensional data of the target oral cavity is obtained based on the plurality of frames of images, and the initial three-dimensional data includes an initial point set of the target oral cavity and three-dimensional coordinate measured values of the target features under the same coordinate system.

A preset model of the intraoral scan post is obtained, and the preset model includes three-dimensional coordinate true values of the target features and a real point set (three-dimensional coordinate true values of various points) of the intraoral scan post under the same coordinate system.

The initial point set of the target oral cavity and the real point set of the intraoral scan post are stitched based on a corresponding relationship between the three-dimensional coordinate measured values of the target features and the true values.

Positioning information of the intraoral scan post is determined based on the stitched real point set of the intraoral scan post, the positioning information of the intraoral scan post is positioning information of the implant, and dental prosthesis design is performed based on the positioning information, such that a designed and manufactured dental prosthesis can be adaptively installed with the implant.

FIG. 5 is a schematic flowchart of a connection method for a scanning apparatus according to an embodiment of the present disclosure. The method may be executed by a connection apparatus for the scanning apparatus. The apparatus may be implemented by software and/or hardware, and is typically integrated in an electronic device. As shown in FIG. 5, the method includes the following:

Step 101: Fixed distances between scanning bodies within an oral cavity region are obtained.

The oral cavity region refers to a tooth region in an oral cavity. Different users have different oral cavity regions. It should be understood that positions of various implants are determined in advance through analysis of the oral cavity region, the scanning bodies are connected with the implants, and therefore, the number of the scanning bodies and the distances between the scanning bodies are fixed, that is, the distances between the scanning bodies are fixed distances.

Specifically, the fixed distances between the scanning bodies are determined based on the distances between the implants.

Step 102: Target auxiliary feature bodies with target lengths are determined based on the fixed distances, such that the scanning bodies are connected based on the target auxiliary feature bodies within the oral cavity region.

In this embodiment of the present disclosure, the scan post apparatus includes a scanning body and an auxiliary feature body. Based on the foregoing description of the scan post apparatus, the scanning body and the auxiliary feature body may be connected in a split manner, the shape and length of the auxiliary feature body may be flexibly set, and therefore target auxiliary feature bodies with target lengths may be selected according to fixed distances between scanning bodies. For example, the fixed distance between the two scanning bodies is 5 mm, and therefore a target auxiliary feature body with the target length of 2 mm and a target auxiliary feature body with the target length of 3 mm may be selected to establish connection between the two scanning bodies.

As an example scenario, the fixed distances between the scanning bodies are determined in advance according to the preset distances between the implants. For example, the fixed distance between scanning bodies A and B is 5 mm, and the fixed distance between scanning bodies C and D is 3 mm. Additionally, target lengths corresponding to various auxiliary feature bodies are preset and stored in advance. For example, a target auxiliary feature body a1 is 2 mm, and a target auxiliary feature body a2 is 3 mm, thereby determining that the scanning bodies A and B are matched with the target auxiliary feature bodies a1 and a2, then connecting the target auxiliary feature bodies a1 and a2 between the scanning bodies A and B, matching the target auxiliary feature body a2 between the scanning bodies C and D, and connecting the other target auxiliary feature body identified as a2 between the scanning bodies C and D.

In this embodiment of the present disclosure, when a total length of the distance between any two of the target auxiliary feature bodies is greater than the fixed distance, the any two of the target auxiliary feature bodies are connected according to a preset connection mode.

Specifically, when the stored total length of the distance between the two target auxiliary feature bodies is greater than the fixed distance, the scanning bodies in this embodiment of the present disclosure may be designed in a non-anti-rotation manner, and may rotate, and therefore any two of the target auxiliary feature bodies may be connected according to the preset connection mode, such as lap joint, thereby further improving flexibility in the implanting and scanning scenario while ensuring the accuracy of intraoral scanning.

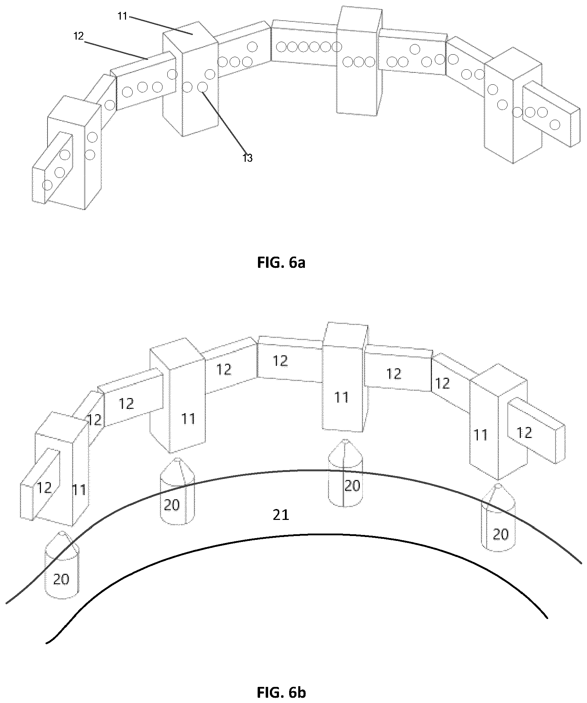

As an example scenario, as shown in FIG. 6a, two scanning bodies are connected through two target auxiliary feature bodies, thereby achieving connection of a plurality of scanning apparatuses, and ensuring that the scanning apparatuses can maintain a relative positional relationship. After intraoral installation of scan posts is finished, a rigid body can be formed, which cannot deform due to deformation of soft tissues such as gums, thereby improving accuracy of intraoral scanning. The shapes of the plurality of scanning apparatuses are preferably different to facilitate scanner recognition. If the plurality of scanning apparatuses have the same shape, the difficulty of an algorithm is increased. As shown in FIG. 6b, the positions of various implants 20 are determined in advance through analysis of an oral cavity region, and scanning bodies 11 are connected with the implants 20, and are in one-to-one correspondence in position.

According to the solution for connecting the scanning apparatuses provided by this embodiment of the present disclosure, the fixed distances between the scanning bodies within the oral cavity region are obtained, and the target auxiliary feature bodies with the target lengths are determined based on the fixed distances, such that the scanning bodies are connected based on the target auxiliary feature bodies within the oral cavity region. By adopting the above technical solution, the intraoral scanner can directly obtain scan post data and feature data through intraoral scanning, thereby improving scanning accuracy of the scan post.

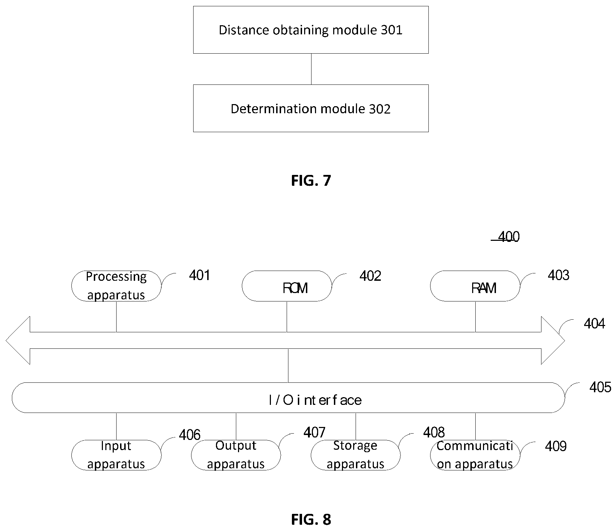

FIG. 7 is a schematic structural diagram of a connection apparatus for a scanning apparatus according to an embodiment of the present disclosure. The apparatus may be implemented by software and/or hardware, and is typically integrated in an electronic device. As shown in FIG. 7, the apparatus includes:
a distance obtaining component 301, configured to obtain fixed distances between scanning bodies within an oral cavity region; and
a determination component 302, configured to determine target auxiliary feature bodies with target lengths based on the fixed distances so as to connect the scanning bodies based on the target auxiliary feature bodies within the oral cavity region.

Alternatively, the apparatus further includes:
an adjustment component, configured to connect any two of the target auxiliary feature bodies according to a preset connection mode when a total length of a distance between any two of the target auxiliary feature bodies is greater than the fixed distance.

The connection apparatus for the scanning apparatus provided by this embodiment of the present disclosure may execute the connection method for the scanning apparatus provided by any embodiment of the present disclosure, and has the corresponding functional components and beneficial effects for executing the method.

An embodiment of the present disclosure further provides a computer program product including computer programs/instructions. The computer programs/instructions, when executed by a processor, implement the connection method for the scanning apparatus provided by any embodiment of the present disclosure.

FIG. 8 is a schematic structural diagram of an electronic device according to an embodiment of the present disclosure. Specifically referring to FIG. 8 below, which illustrates a schematic structural diagram of an electronic device 400 suitable for implementing an embodiment of the present disclosure. The electronic device 400 in this embodiment of the present disclosure may include, but is not limited to, mobile terminals such as a mobile phone, a notebook computer, a digital radio receiver, a personal digital assistant (PDA), a portable Android device (PAD), a portable media player (PMP), and a vehicle-mounted terminal (e.g., a vehicle-mounted navigation terminal), and fixed terminals such as a digital TV and a desktop computer. The electronic device shown in FIG. 8 is merely an example, which should not impose any limitations on functions and application ranges of this embodiment of the present disclosure.

As shown in FIG. 8, the electronic device 400 may include a processing apparatus (e.g., a central processing unit and a graphics processing unit) 401, which may execute various appropriate actions and processing according to programs stored on a read only memory (ROM) 402 or loaded from a storage apparatus 408 into a random access memory (RAM) 403. The RAM 403 further stores various programs and data required for the operation of the electronic device 400. The processing apparatus 401, the ROM 402, and the RAM 403 are connected to one another through a bus 404. An input/output (I/O) interface 405 is also connected to the bus 404.

Typically, the following apparatuses may be connected to the I/O interface 405: an input apparatus 406, including, for example, a touchscreen, a touchpad, a keyboard, a mouse, a camera, a microphone, an accelerometer, and a gyroscope; an output apparatus 407, including, for example, a liquid crystal display (LCD), a speaker, and a vibrator; the storage apparatus 408, including, for example, a magnetic tape and a hard drive; and a communication apparatus 409. The communication apparatus 409 may allow the electronic device 400 to be in wireless or wired communication with other devices for data exchange. Although FIG. 8 illustrates the electronic device 400 with various apparatuses, it should be understood that it is not necessary to implement or have all the shown apparatuses. Alternatively, more or fewer apparatuses may be implemented or provided.

Particularly, the foregoing process described with reference to the flowchart according to the embodiments of the present disclosure may be implemented as a computer software program. For example, an embodiment of the present disclosure includes a computer program product including a computer program carried on a non-transitory computer-readable medium. The computer program includes program code for executing the method shown in the flowchart. In this embodiment, the computer program may be downloaded and installed from the network by the communication apparatus 409, or installed from the storage apparatus 408, or installed from the ROM 402. The computer program, when executed by the processing apparatus 401, executes the above functions limited in the connection method for the scanning apparatus in this embodiment of the present disclosure.

It should be noted that the computer-readable medium in the present disclosure may be a computer-readable signal medium, or a computer-readable storage medium, or any combination of the above. For example, the computer-readable storage medium may include, but is not limited to: electrical, magnetic, optical, electromagnetic, infrared, or semiconductor systems, apparatuses, or devices, or any combination thereof. More specific examples of the computer-readable storage medium may include, but are not limited to: an electrical connection with one or more wires, a portable computer disk, a hard disk, a random access memory (RAM), a read only memory (ROM), an erasable programmable read only memory (EPROM or a flash memory), fiber optics, a portable compact disc read only memory (CD-ROM), an optical storage device, a magnetic storage device, or any proper combination of the above. In the present disclosure, the computer-readable storage medium may be any tangible medium including or storing a program, and the program may be used by an instruction execution system, apparatus, or device, or used in conjunction with the instruction execution system, apparatus, or device. However, in the present disclosure, the computer-readable signal medium may include data signals propagated in a baseband or propagated as a part of a carrier wave, which carry computer-readable program code. The propagated data signals may have a plurality of forms, including, but not limited to, electromagnetic signals, optical signals, or any proper combination of the above. The computer-readable signal medium may also be any computer-readable medium other than the computer-readable storage medium. The computer-readable signal medium may send, propagate, or transmit the program used by the instruction execution system, apparatus, or device, or used in conjunction with the instruction execution system, apparatus, or device. The program code included in the computer-readable medium may be transmitted by any proper medium including, but not limited to, a wire, an optical cable, radio frequency (RF), etc., or any proper combination of the above.

In some implementations, a client and a server may communicate using any currently known or future-developed network protocols such as a hyper text transfer protocol (HTTP), and may also be in communication connection with digital data in any form or medium (e.g., a communication network). Examples of the communication network include a local area network ("LAN"), a wide area network ("WAN"), Internet work (e.g., Internet), a peer-to-peer network (e.g., an ad hoc peer-to-peer network), and any currently known or future-developed networks.

The computer-readable medium may be included in the above electronic device; and may separately exist without being assembled in the electronic device.

The computer-readable medium carries one or more programs. The one or more programs, when executed by the electronic device, enable the electronic device to: receive an information display trigger operation of a user in a video playing process; obtain at least two pieces of target information associated with a video; display first target information from the at least two pieces of target information in an information display region of a video playing page, where the size of the information display region is less than that of the playing page; and receive a first switching trigger operation of the user, and switch the first target information displayed in the information display region into second target information from the at least two pieces of target information.

The computer program code for executing the operations of the present disclosure may be written in one or more programming languages or a combination thereof. The programming languages include, but are not limited to, object-oriented programming languages such as Java, Smalltalk, C++, as well as conventional procedural programming languages such as "C" or similar programming languages. The program code may be executed entirely on a user computer, executed partially on the user computer, executed as a standalone software package, executed partially on the user computer and partially on a remote computer, or executed entirely on the remote computer or a server. In the case of involving the remote computer, the remote computer may be connected to the user computer via any type of network, including a local area network (LAN) or wide area network (WAN), or may be connected to an external computer (e.g., utilizing an Internet service provider for Internet connectivity).

The flowcharts and block diagrams in the accompanying drawings illustrate system architectures, functions, and operations possibly implemented by the system, method and computer program product according to the various embodiments of the present disclosure. In this regard, each block in the flowcharts or block diagrams may represent a module, a program segment, or a portion of code, and the module, program segment, or portion of code includes one or more executable instructions for implementing specified logical functions. It should be noted that in some alternative implementations, functions marked in the blocks may also occur in an order different from that marked in the accompanying drawings. For example, two consecutively-shown blocks may actually be executed in parallel basically, but sometimes may also be executed in a reverse order, which depends on involved functions. It should be further noted that each block in the block diagrams and/or flowcharts as well as a combination of the blocks in the block diagrams and/or flowcharts may be implemented by using a dedicated hardware-based system that executes specified functions or operations, or using a combination of special hardware and computer instructions.

The units described in the embodiments of the present disclosure may be implemented through software or hardware. The name of the unit does not limit the unit in certain cases.

The functions described above in this specification may be at least partially executed by one or more hardware logic components. For example, exemplary hardware logic components that can be used include, but are not limited to, a field-programmable gate array (FPGA), an application specific integrated circuit (ASIC), an application specific standard part (ASSP), a system on chip (SOC), a complex programmable logic device (CPLD), etc.

In the context of the present disclosure, a machine-readable medium may be a tangible medium that may contain or store a program, and the program may be used by the instruction execution system, apparatus, or device, or used in conjunction with the instruction execution system, apparatus, or device. The machine-readable medium may be a machine-readable signal medium or a machine-readable storage medium. The machine-readable medium may include, but is not limited to: electronic, magnetic, optical, electromagnetic, infrared, or semiconductor systems, apparatuses, or devices, or any proper combination of the above. More specific examples of the machine-readable storage medium may include: an electrical connection based on one or more wires, a portable computer disk, a hard drive, a random access memory (RAM), a read only memory (ROM), an erasable programmable read only memory (EPROM or a flash memory), fiber optics, a portable compact disc read only memory (CD-ROM), an optical storage device, a magnetic storage device, or any proper combination of the above.

According to one or more embodiments of the present disclosure, the present disclosure provides an electronic device, including:
a processor; and
a memory configured to store executable instructions of the processor.

The processor is configured to read the executable instructions from the memory, and execute the instructions to implement any one of the methods for connecting the scanning apparatuses provided by the present disclosure.

According to one or more embodiments of the present disclosure, the present disclosure provides a computer-readable storage medium. The storage medium stores a computer program. The computer program is configured to execute any one of the methods for connecting the scanning apparatuses provided by the present disclosure.

It should be noted that herein, relational terms such as "first" and "second" are used only to distinguish one entity or operation from another and do not necessarily require or imply any actual relationship or order between these entities or operations. In addition, terms "comprise", "include" or any other variations thereof are intended to cover non-exclusive inclusion, and therefore a process, a method, an object, or a device including a series of elements not only includes those elements but also includes other elements not clearly listed, or further includes inherent elements for the process, the method, the object, or the device. In the absence of further restrictions, an element specified by the phrase "including a..." does not exclude the existence of other identical elements in the process, method, product, or, device that includes the element.

The above contents are merely specific implementations of the present disclosure, such that those skilled in the art can understand or implement the present disclosure. More modifications for these embodiments are apparent to those skilled in the art, and general principles defined herein may be implemented in other embodiments without departing from the spirit or scope of the present disclosure. Thus, the present disclosure will not be limited by these embodiments shown herein but is required to conform to a widest scope consistent with the principles and novel characteristics disclosed herein.

### Industrial applicability

The scanning apparatus and the connection method thereof provided by the present disclosure simplify a using mode, require less external assistance, and can improve scanning and implanting accuracy during positioning based on coordinate information of the auxiliary feature points, thereby further increasing a utilization rate of an intraoral scanner in implant cases and having strong industrial applicability.

## Claims

1. A scanning apparatus, comprising:
a scanning body; and
an auxiliary feature body connected with the scanning body,
wherein auxiliary feature points are arranged on the scanning body and/or the auxiliary feature body.

2. The scanning apparatus as claimed in claim 1, wherein
the scanning body is provided with an adjustable-angle connection structure connected with the auxiliary feature body.

3. The scanning apparatus as claimed in claim 1 or 2, wherein
the auxiliary feature body is connected with the scanning body through threads; and/or,
the auxiliary feature body is connected with the scanning body through a latch; and/or,
the auxiliary feature body is fixedly connected with the scanning body through a collar.

4. The scanning apparatus as claimed in claims 1 to 3, wherein the auxiliary feature points comprises:
spherical; and/or,
codes; and/or,
mark points.

5. The scanning apparatus as claimed in any one of claims 1 to 4, wherein the auxiliary feature body comprises:
drum-shaped; and/or,
rectangular; and/or,
conical.

6. The scanning apparatus as claimed in claims 1 to 5, wherein
the scanning body is provided with a non-anti-rotation structure connected with an implant.

7. The scanning apparatus as claimed in claims 1 to 6, wherein
the auxiliary feature body extends laterally outward from a scan post to be matched with an auxiliary feature body on an adjacent intraoral scan post, allowing target shape feature components on the two auxiliary feature bodies to be continuously distributed.

8. The scanning apparatus as claimed in any one of claims 1 to 7, wherein
the two scanning bodies corresponding to the two auxiliary feature bodies are connected by connecting the two auxiliary feature bodies; and
a distribution distance between the auxiliary feature points set on the two auxiliary feature bodies is less than a preset distance threshold.

9. A connection method for the scanning apparatus as claimed in any one of claims 1 to 8, comprising:
obtaining fixed distances between scanning bodies within an oral cavity region; and
determining target auxiliary feature bodies with target lengths based on the fixed distances so as to connect the scanning bodies based on the target auxiliary feature bodies within the oral cavity region.

10. The connection method for the scanning apparatus as claimed in claim 9, further comprising:
connecting any two of the target auxiliary feature bodies according to a preset connection mode when a total length of a distance between any two of the target auxiliary feature bodies is greater than the fixed distance.

11. A connection apparatus for the scanning apparatus as claimed in any one of claims 1 to 8, comprising:
a distance obtaining component, configured to obtain fixed distances between scanning bodies within an oral cavity region; and
a determination component, configured to determine target auxiliary feature bodies with target lengths based on the fixed distances so as to connect the scanning bodies based on the target auxiliary feature bodies within the oral cavity region.

12. An electronic device, comprising
a processor; and
a memory configured to store executable instructions of the processor,
the processor being configured to read the executable instructions from the memory, and execute the instructions to implement the connection method for the scanning apparatus as claimed in claim 10 or 11.

13. A computer-readable storage medium, wherein the storage medium has a computer program stored therein, and the computer program is configured to execute the connection method for the scanning apparatus as claimed in claim 10 or 11.
